Europäisches Patentamt

European Patent Office

Office européen des brevets

(11) Numéro de publication: **0 112 423**
**A1**

## (12) DEMANDE DE BREVET EUROPEEN

(21) Numéro de dépôt: **82402322.0**

(51) Int. Cl.³: **A 61 F 1/03**

(22) Date de dépôt: **17.12.82**

(43) Date de publication de la demande: **04.07.84**
**Bulletin 84/27**

(71) Demandeur: **Bréard, Francis Henri, 25 rue de Coulmiers, F-75014 Paris (FR)**

(72) Inventeur: **Bréard, Francis Henri, 25 rue de Coulmiers, F-75014 Paris (FR)**

(74) Mandataire: **Chauchard, Robert et al, c/o Cabinet Malémont 42, avenue du Président Wilson, F-75116 Paris (FR)**

(84) Etats contractants désignés: **AT BE CH DE FR GB IT LI LU NL SE**

(54) Prothèse articulaire, et notamment prothèse fémorale, à tige intramédullaire auto-blocante en forme de coin.

(57) La tige d'ancrage intramédullaire (1) présente deux faces (6, 7) de largeur progressivement décroissante en direction de l'extrémité libre (10) de la tige et deux chants bombés (8, 9) qui, au moins en partie, sont destinés à venir s'appuyer contre la paroi du canal médullaire.

Les deux chants bombés (8, 9) de la tige intramédullaire (1) ont, au moins dans leur zone d'appui (AB, CD), une section droite sensiblement en forme d'arc de cercle ($S_1$ à $S_4$ et $T_2$ à $T_4$) dont le rayon de courbure ($r_1$ à $r_4$) décroît en direction de l'extrémité libre de la tige (1).

De préférence, sur toute la longueur de la tige (1) et au moins dans les zones d'appui (AB, CD) de ses chants bombés (8, 9), la section ($S_1$ à $S_4$ et $T_2$ à $T_4$) de ces derniers présente sensiblement la forme d'un arc de cercle centré sur l'axe principal (X) de la tige.

EP 0 112 423 A1

- 1 -

Prothèse articulaire, et notamment prothèse fémorale, à tige intramédullaire auto-blocante en forme de coin.

La présente invention concerne une prothèse articulaire, et notamment une prothèse fémorale, du type comprenant un élément prothétique d'articulation, tel qu'une tête sphérique, porté par une tige d'ancrage intramédullaire emboîtable dans le canal médullaire préalablement évidé de l'os d'implantation, cette tige présentant deux faces dont la largeur diminue progressivement en direction de son extrémité libre et deux chants bombés qui, sur au moins une partie de leur longueur, sont destinés à venir s'appuyer contre la paroi intérieure du canal médullaire.

La mise en place dans le fémur d'une prothèse fémorale présentant cette configuration, consiste, dans un premier temps, à emboîter sa tige intramédullaire dans le canal médullaire doté d'une forme générale conique à sa partie basse. En venant s'appuyer par ses deux chants bombés contre la paroi conique de ce canal, la tige intramédullaire est bloquée à l'intérieur du fémur, l'ancrage définitif de la prothèse étant ensuite réalisé par une adjonction de ciment acrylique de chaque côté de la tige.

Ainsi, dans le cas d'une dégradation de la liaison cimentée, la tige reste immobile à l'intérieur du canal et, si en plus l'état de surface de la paroi du canal se détériore, elle s'enfonce par gravité et se rebloque automatiquement.

C'est pourquoi, on désigne communément ces prothèses sous l'expression "prothèses à tige intramédullaire auto-blocante" par opposition aux prothèses classiques à cimentage total.

On sait d'ailleurs que, pour reconstituer les cols de fémur, dans le cadre des opérations de remplacement total ou partiel de l'articulation de la hanche, les chirurgiens prothésistes utilisent de plus en plus de telles prothèses "auto-blocantes" à la place des prothèses classiques qui présentent l'inconvénient que, dans le cas d'une dégradation de la liaison cimentée, leur tige remue à l'intérieur du canal médullaire en provoquant des douleurs de cuisse très vives, voire même des blessures de l'os.

Toutefois, l'emploi des prothèses "auto-blocantes", actuellement mises sur

le marché, a fait apparaître à l'usage d'autres inconvénients résultant essentiellement de la configuration donnée aux chants bombés de leur tige intramédullaire.

En effet, dans ces prothèses "auto-blocantes" connues, les chants bombés de la tige sont, pour des raisons de simplification d'usinage, réalisés avec un rayon de courbure constant d'une extrémité à l'autre de la tige. Il s'ensuit bien évidemment que ces chants bombés ne viennent pas en contact total avec la paroi du canal médullaire et que la pression d'appui qu'ils exercent contre celle-ci n'est pas uniformément répartie.

C'est ainsi qu'à l'entrée du canal médullaire, le rayon de courbure du chant interne est en général inférieur à celui de la section correspondante du canal si bien qu'il s'exerce contre la paroi de ce dernier une hyperpression concentrée en un seul point, conduisant bien souvent à un éclatement de l'éperon de Merckel.

Par contre, à proximité du fond du canal, le rayon de courbure des deux chants de la tige est souvent supérieur à celui de la section correspondante du canal et l'on a alors un contact de pression anguleux des quatre arêtes de la tige contre la paroi du canal, qui fait naître des douleurs de cuisse très vives.

La présente invention se propose de remédier à ces inconvénients et, pour ce faire, elle a pour objet une prothèse articulaire, et notamment une prothèse fémorale, du type spécifié en préambule, qui se caractérise en ce que les deux chants bombés de sa tige intramédullaire ont, au moins dans leur zone d'appui, une section sensiblement en forme d'arc de cercle dont le rayon de courbure décroît en direction de l'extrémité libre de la tige.

Par ailleurs, aux deux extrémités des zones d'appui des chants bombés de la tige, la section droite de ces derniers présente sensiblement la forme d'un arc de cercle centré sur l'axe principal de la tige.

Dans un mode préféré de réalisation de l'invention, sur toute la longueur de la tige et au moins dans les zones d'appui de ses chants bombés, la section de ces derniers présente sensiblement la forme d'un arc de cercle centré sur l'axe principal de la tige.

- 3 -

Grâce à ces dispositions, le rayon de courbure des sections des chants bombés de la tige varie dans le même sens que celui de la section du canal médullaire de l'os . Bien plus, après emboîtement de la tige intramédullaire.          on obtient, sur toute la longueur des zones d'appui des chants bombés, une congruence quasiment parfaite entre l'arc de courbure de ces derniers et celui de la paroi du canal.

Par comparaison avec les prothèses "auto-blocantes" actuellement connues, la prothèse selon l'invention permet donc de réaliser une meilleure répartition de la pression d'appui de la tige intramédullaire contre la paroi intérieure du canal médullaire. Il en résulte que les hyperpressions d'appui apparaissant avec l'emploi des prothèses "auto-blocantes" connues sont éliminées sur toute la hauteur du canal médullaire et plus particulièrement au voisinage de son entrée et de son fond, là où elles sont le plus préjudiciables.

Enfin pour renforcer cet effet de congruence, on donne à la tige de la prothèse  objet de l'invention, une largeur de chant qui diminue progressivement en direction de son extrémité libre.

Un mode de réalisation de la présente invention va être décrit ci-après, à titre d'exemple non-limitatif, en référence aux dessins annexés dans lesquels :
- la figure 1 est une vue de face d'une prothèse fémorale conforme à l'invention en place dans un fémur ;
- la figure 2 est une vue latérale de cette prothèse sans sa tête sphérique ;
- la figure 3 est une vue schématique latérale de la tige de cette prothèse, qui illustre plus spécialement l'évolution de l'arc de courbure de son chant bombé interne.
- les figures 4 et 5 a  à 5 d  sont des schémas explicatifs de la manière dont sont réalisés les chants bombés de cette tige ;
- les figures 6, 7, 8 et 9 sont des vues en coupe effectuées respectivement selon les lignes VI-VI, VII-VII, VIII-VIII et IX-IX de la figure 1, dans lesquelles le fémur est simplement figuré par son canal médullaire, et,
- les figures 10 et 11 sont, à titre de comparaison, des vues en coupe analogues respectivement aux figures 6 et 9 mais se rapportant à une pro- thèse "auto-blocante" de type connu.

- 4 -

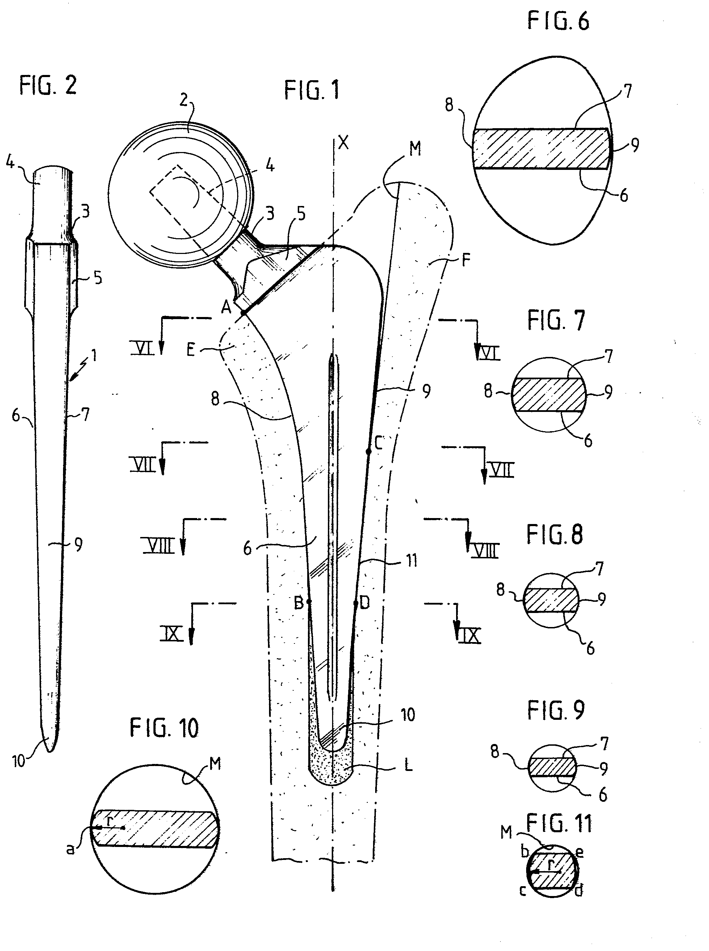

La prothèse fémorale selon l'invention, représentée sur les figures 1, 2 et 6 à 9, comprend une tige intramédullaire, désignée dans son ensemble par 1, et une tête 2 de forme générale sphérique portée par un col 3 solidaire de la tige 1 et incliné par rapport à l'axe principal X de cette dernière. Plus précisément, la tête 2 est emmanchée à force sur un prolongement 4 légèrement conique du col 3 et ce dernier se rattache à la tige 1 par une partie épaissie 5.

En vue de l'ancrage de la prothèse dans le fémur F à reconstituer, la tige intramédullaire 1 est, dans un premier temps, emboîtée dans le canal médullaire M, préalablement évidé, de ce dernier. Ce canal médullaire M est divisé, dans le sens de sa hauteur, en deux parties, séparées sur la figure 1 par la ligne de coupe VII-VII.

La partie basse du canal M présente une paroi conique de section sensiblement circulaire (délimitée par les lignes de coupe VII-VII et IX-IX sur la figure 1), qui se prolonge par une paroi cylindrique jusqu'au fond du canal. Quant à la partie haute du canal M, elle est formée, du côté interne du fémur, c'est-à-dire entièrement à gauche du plan longitudinal médian du fémur (matérialisé par l'axe X sur la figure 1), d'une paroi incurvée en direction de l'éperon de Merckel E et ayant sensiblement une section semi-circulaire (voir figure 6). La forme de la paroi de la partie haute du canal M, du côté externe du fémur, est sans importance pour la suite de la description.

De manière connue en soi, la tige intramédullaire 1 présente deux faces planes 6 et 7 dont la largeur diminue progressivement en direction de l'extrémité libre de la tige, conformément à la variation du diamètre du canal médullaire M. La tige 1 comporte en outre deux chants bombés 8 et 9 par lesquels elle vient s'appuyer contre la paroi du canal médullaire.

On fera observer ici, en référence à la figure 1, que la tige 1 s'appuie par ses deux chants bombés 8, 9, contre la paroi conique de la partie basse du canal médullaire M, alors que, seul, son chant interne 8, qui est d'ailleurs incurvé à cet effet, exerce une pression contre la paroi de la partie haute du canal, au niveau de l'éperon de Merckel E du fémur. On peut ainsi définir, sur chacun des chants bombés 8 et 9, leur zone d'appui effective qui, sur la figure 1, sont matérialisées par les segments de courbe AB et CD.

On notera encore que la tige intramédullaire 1 se termine par une pointe 10, qui après l'emboîtement de la tige, vient se planter dans un lit L de ciment acrylique préalablement déposé dans le fond du canal médullaire. En outre, une fine rainure 11 est prévue sur chacune des faces 6, 7 de la tige 1 pour assurer une adhérence sur cette dernière du ciment que l'on coule dans la cavité médullaire M en vue de l'ancrage définitif de la prothèse dans le fémur.

Il est en outre à remarquer qu'à sa partie basse, c'est-à-dire en dessous de la ligne de coupe VII-VII de la figure 1, la tige 1 est parfaitement symétrique vis-à-vis de son axe principal X qui, après emboîtement de la tige, est confondu avec l'axe longitudinal médian du fémur.

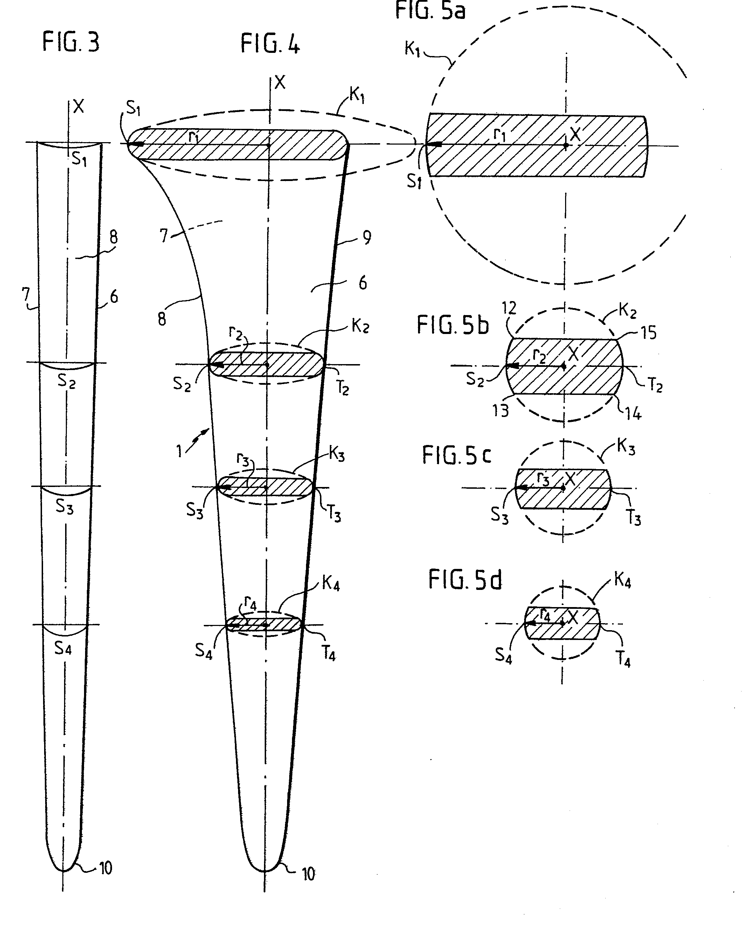

On sait que, dans les prothèses de ce type, actuellement disponibles sur le marché, les chants bombés de la tige intramédullaire présentent un rayon de courbure r constant, comme on peut le voir sur les figures 10 et 11. Ceci a pour conséquence que la pression exercée par la tige sur la paroi du canal médullaire M n'est pas uniformément répartie.

C'est ainsi qu'à l'entrée du canal médullaire M, le chant bombé intérieur peut exercer, comme le montre la figure 10, une hyperpression presque entièrement concentrée en un seul point a, cette hyperpression provoquant généralement un éclatement de l'éperon de Merckel.

Par contre, lorsqu'on se rapproche du fond du canal M, on peut se heurter à l'inconvénient illustré par la figure 11, à savoir que seules les arêtes de la tige b, c, d et e s'appuient contre la paroi du canal en faisant naître des douleurs très vives dans la cuisse.

La prothèse selon l'invention remédie à ces inconvénients comme on va le voir ci-après en se référant aux figures 3 à 9.

Selon la caractéristique première de l'invention, les deux chants bombés 8 et 9 de la tige intramédullaire 1 présentent, dans leurs zones d'appui AB et CD, une section droite sensiblement en forme d'arc de cercle dont le rayon de courbure décroît en direction de l'extrémité libre de la tige. En d'autres termes, la courbure des chants bombés va en s'accentuant vers le bas. C'est ce qui ressort clairement des figures 4 et 5 à 5 d qui montrent la décroissance du rayon de courbure $r_1$ à $r_4$ des sections des chants bombés et de la figure 3 qui représente l'évolution de l'arc de courbure $S_1$ à $S_4$ de la section du chant interne 8.

Les figures 4 et 5 à 5 d illustrent également la manière dont sont définies les sections droites des zones d'appui AB et CD des chants bombés 8, 9, de la tige 1.

Comme on peut le voir, en tout point des zones d'appui AB et CD des chants bombés 8 et 9 et notamment aux extrémités de celles-ci, ces sections sont sensiblement formées par l'arc $S_1$ à $S_4$ et $T_2$ à $T_4$ d'un cercle $K_1$ à $K_4$ centré sur l'axe principal X de la tige 1 et passant par les deux arêtes 12,13 ou 14,15 de cette dernière qui relient le chant considéré 8 ou 9 aux deux faces 6 et 7.

Pour la partie d'extrémité de la tige 1, symétrique par rapport à l'axe X (située en dessous de la ligne de coupe VII-VII de la figure 1), cela revient à dire que les arcs $S_2$, $S_3$ ou $S_4$ et $T_2$, $T_3$ ou $T_4$ d'un même plan de coupe, sont ceux d'un même cercle circonscrit au rectangle défini dans ce plan par les deux faces 6 et 7 de la tige 1, comme on peut le voir sur les figures 5 b à 5 d.

La figure 5 a montre par ailleurs que la forme de la section du chant bombé externe 9, dans la partie haute de la tige peut être quelconque, dans la mesure où ce chant externe ne vient pas, dans cette partie de la tige, en appui contre la paroi du canal médullaire.

Ainsi, comme cela ressort clairement des figures 6 à 9, le degré de courbure des sections des chants bombés 8, 9 de la tige, correspond sensiblement, en tout point des zones d'appui AB et CD, à celui de la paroi conique du canal médullaire. On obtient ainsi, sur toute la hauteur du canal médullaire, une congruence quasiment parfaite entre la paroi intérieure de ce dernier et les chants bombés 8, 9 de la tige 1. La pression d'appui de la tige 1 est ainsi uniformément répartie sur la paroi du canal médullaire avec pour conséquence que les problèmes d'hyperpression, exposés ci-dessus en référence aux figures 10 et 11, sont totalement éliminés.

Ce résultat est renforcé par le fait que, selon l'invention, la largeur de chant de la tige 1, c'est-à-dire la distance séparant ses faces 6 et 7, diminue progressivement en direction de l'extrémité libre de la tige, comme on peut le voir sur la figure 2.

Comme on l'aura compris, la configuration particulière décrite ci-dessus, que l'on donne aux chants bombés 8, 9 de la tige 1, n'est nécessaire qu'à l'intérieur des zones d'appui AB et CD de ces derniers. Il est évident toutefois que, pour des raisons pratiques de fabrication et parce qu'il est difficile de déterminer à l'avance la longueur précise de ces zones d'appui, les chants 8, 9 peuvent être usinés suivant cette configuration sur toute la hauteur de la tige 1.

On précisera encore que, dans la mesure où le canal médullaire du fémur ne présente pas une section rigoureusement circulaire ni une surface de paroi parfaitement lisse, une certaine tolérance d'usinage est admise pour la réalisation des chants bombés.

Enfin, il va de soi que l'on peut mettre à la disposition des chirurgiens prothésistes toute une gamme de prothèses selon l'invention, conformées aux dimensions du canal médullaire.

0112423

REVENDICATIONS

1. Prothèse articulaire, et notamment prothèse fémorale, du type comprenant un élément prothètique d'articulation, tel qu'une tête sphérique (2), porté par une tige d'ancrage intramédullaire (1) emboîtable dans le canal médullaire préalablement évidé de l'os d'implantation, cette tige (1) présentant deux faces (6, 7) dont la largeur diminue progressivement en direction de son extrémité libre et deux chants bombés (8, 9) qui, sur au moins une partie de leur longueur, sont destinés à venir s'appuyer contre la paroi intérieure du canal médullaire, caractérisée en ce que les deux chants bombés (8, 9) de la tige intramédullaire (1) ont, au moins dans leur zone d'appui (AB, CD), une section droite sensiblement en forme d'arc de cercle ($S_1$ à $S_4$ et $T_2$ à $T_4$) dont le rayon de courbure ($r_1$ à $r_4$) décroît en direction de l'extrémité libre de la tige (1).

2. Prothèse articulaire selon la revendication 1, caractérisée en ce que, aux deux extrémités des zones d'appui (AB, CD) des chants bombés (8, 9) de la tige (1), la section droite ($S_1$, $S_4$ et $T_2$, $T_4$) de ces derniers présente sensiblement la forme d'un arc de cercle centré sur l'axe principal (X) de la tige.

3. Prothèse selon la revendication 2, caractérisée en ce que, sur toute la longueur de la tige (1) et au moins dans les zones d'appui (AB, CD) de ses chants bombés (8, 9), la section ($S_1$ à $S_4$ et $T_2$ à $T_4$) de ces derniers présente sensiblement la forme d'un arc de cercle centré sur l'axe principal (X) de la tige.

4. Prothèse selon la revendication 1, 2 ou 3, caractérisée en ce que la tige (1) présente une largeur de chant qui diminue progressivement en direction de son extrémité libre.

0112423

FIG. 6
FIG. 2
FIG. 1
FIG. 7
FIG. 8
FIG. 9
FIG. 10
FIG. 11

0112423

2/2

FIG. 3

FIG. 4

FIG. 5a

FIG. 5b

FIG. 5c

FIG. 5d

# 0112423

Office européen
des brevets

**RAPPORT DE RECHERCHE EUROPEENNE**

Numéro de la demande

EP   82 40 2322

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| Y | FR-A-2 380 021  (RICHARDS MANUFACTURING CO. INC.) * Page 5, lignes 3-17; figures 1-7 * | 1 | A 61 F    1/03 |
| | --- | | |
| Y | DE-A-2 331 728  (RITTER et al.) * Page 5, lignes 3-8; figures 2, 3 * | 1 | |
| | --- | | |
| Y | GB-A-2 052 999  (STE EUROPEENNE DE PROPULSION) * Figure 4 * | 1 | |
| | --- | | |
| A | FR-A-2 404 429  (BREARD) | | |
| | --- | | |
| A | FR-A-2 111 202  (MATHYS) | | DOMAINES TECHNIQUES RECHERCHES (Int. Cl. ³) |
| | --- | | |
| A | GB-A-2 096 003  (NEW YORK STY FOR THE RELIEF OF THE RUPTURED AND CRIPPLED, MAINTAINING THE HOSPITAL FOR SPECIAL SURGERY) | | A 61 F    1/00 |
| | --- | | |
| A | US-A-2 719 522  (HUDACK) | | |
| | ----- | | |

Le présent rapport de recherche a été établi pour toutes les revendications

| Lieu de la recherche | Date d'achèvement de la recherche | Examinateur |
|---|---|---|
| BERLIN | 22-07-1983 | KANAL P K |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82